# EUROPÄISCHE PATENTSCHRIFT

(11) **EP 3 393 578 B1**
(45) Veröffentlichungstag und Bekanntmachung des Hinweises auf die Patenterteilung: **07.06.2023**
(21) Anmeldenummer: 16829058.3
(22) Anmeldetag: 07.12.2016
(51) Int. Cl.: A61N 2/00, A61N 2/02

(54) **VORRICHTUNG ZUR ERZEUGUNG VON MAGNETISCHEN WECHSELFELDERN ZUM INDUZIEREN VON WIRBELSTRÖMEN IN EINEM ORGANISMUS**
DEVICE FOR GENERATING ALTERNATING MAGNETIC FIELDS FOR INDUCING EDDY CURRENTS IN AN ORGANISM
DISPOSITIF SERVANT À GÉNÉRER DES CHAMPS MAGNÉTIQUES ALTERNATIFS AFIN D'INDUIRE DES COURANTS DE FOUCAULT DANS UN ORGANISME

(30) Priorität: 23.12.2015 AT 8202015; 20.09.2016 AT 4302016
(43) Veröffentlichungstag der Anmeldung: 31.10.2018
(73) Patentinhaber: Cleanhearing Inc., Lewes, Delaware 19958 (US)
(72) Erfinder: Neuwirth, Gerald, 9800 Spittal/Drau (AU)
(74) Vertreter: Miksovsky, Alexander
(86) Internationale Anmeldenummer: PCT/AT2016/000098
(87) Internationale Veröffentlichungsnummer: WO 2017/106884

(56) Entgegenhaltungen:
- WO-A1-99/04854
- WO-A1-2011/009807
- WO-A1-2012/168543
- DE-A1- 10 310 084
- JP-A- 2006 254 524
- KR-A- 20100 078 190
- KR-A- 20140 108 030
- US-A1- 2015 148 586

## Beschreibung

Die vorliegende Erfindung betrifft eine Vorrichtung zur Erzeugung von magnetischen Wechselfeldern. Die vorliegende Erfindung betrifft insbesondere eine Vorrichtung zur Erzeugung von magnetischen Wechselfeldern zum Induzieren von Wirbelströmen in einem Organismus zur Behandlung insbesondere von Tinnitus, umfassend
- einen Kopfteil,
- eine Stromquelle,
- eine Steuerung,
- eine oder mehrere Spulen.

Die vorliegende Erfindung bezieht sich auf eine Vorrichtung zur Erzeugung von magnetischen Wechselfeldern zum Induzieren von Wirbelströmen in einem Organismus sowie auf eine Verwendung einer derartigen Vorrichtung. Die Vorrichtung zur Erzeugung und Abgabe von magnetischen Wechselfeldern ist mit einer einstellbaren, niedrigen Frequenz mit Impulsen, steilen Flanken und Oberwellenanteilen ausgebildet.

Es sind unterschiedliche Vorrichtungen zur Erzeugung und Abgabe von elektromagnetischen Wechselfeldern zur Behandlung und Untersuchung von Organismen bekannt, wobei allgemein davon auszugehen ist, dass die Eindringtiefe derartiger elektromagnetischer Wechselfelder von der Frequenz abhängt.

WO 2011/009807 beschreibt eine Tinnitus-Therapievorrichtung als Kombination zwischen einem Applikator für niederfrequente elektromagnetische Felder (Therapie) und einer Elektroenzephalografie (EEG) als Quelle für die Auswertung der Auswirkung der Therapie (Diagnose). In der Patentanmeldung WO 2011/009807 sind keine Hinweise vorhanden, über die Art und Weise wie die Eisenkernspulen der Applikatoren gewickelt sind.

WO 1999/04854 beschreibt eine Vorrichtung zur Erzeugung von magnetischen Wechselfeldern zum Induzieren von Wirbelströmen in einem Organismus. In der Patentanmeldung WO 1999/04854 sind wiederum keine Hinweise vorhanden, über die Art und Weise wie die Eisenkernspulen der Applikatoren gewickelt sind.

Weitere ähnliche Vorrichtungen zur Behandlung von Tinnitus der eingangs genannten Art sind beispielsweise der DE 103 10 084 A1 oder der EP 0 537 385 A1 zu entnehmen, wobei wiederum keine Details betreffend eine Wicklung von in derartigen Vorrichtungen zum Einsatz gelangenden Spulen bzw. Solenoiden geoffenbart sind.

Es ist eine Aufgabe dieser Erfindung, eine neue und bessere Vorrichtung vorzuschlagen, welche es ermöglicht, magnetische Wechselfelder zum Induzieren von Wirbelströmen in einem Organismus zur Behandlung insbesondere von Tinnitus wirksamer zu erzeugen.

Zur Lösung dieser Aufgabe ist eine Vorrichtung der eingangs genannten Art im Wesentlichen dadurch gekennzeichnet, dass die wenigstens eine Spule wenigstens zwei Lagen mit unterschiedlicher Wicklung aufweist.

Die vorliegende Erfindung zielt nun darauf ab, eine Vorrichtung zur Erzeugung von magnetischen Wechselfeldern zum Induzieren von Wirbelströmen in einem Organismus zur Verfügung zu stellen, mit welcher in konstruktiv einfacher Weise gezielt und gefahrlos in einem Organismus Wirbelströme erzeugt werden können, um durch die hervorgerufenen Reizungen des vegetativen Nervensystems eine gezielte Beeinflussung von möglichen Ladungsverschiebungen in den Zellmembranen zu bewirken bzw. vorhandene Blockierungen abbauen bzw. mildern zu können. Da magnetische Wechselfelder einer einstellbaren, niedrigen Frequenz von der erfindungsgemäßen Vorrichtung abgegeben werden, gelingt eine einfache Anpassung an die bei unterschiedlichen Organismen unterschiedliche Empfindlichkeit. Dabei wurde festgestellt, dass die Empfindlichkeit bei jener Frequenz maximal ist, welche mit dem EEG- α-Rhythmus der zu behandelnden Person übereinstimmt. Durch die erfindungsgemäß gewählten, geringen Feldstärken ist weiter sichergestellt, dass bei unproblematischer und absolut gefahrloser Handhabung die gewünschten Effekte im Hinblick auf das Induzieren von Wirbelströmen im Organismus erzielbar sind. Dabei werden Impulse in Form von Wellen mit pulsierenden, steilen Flanken mit Oberwellenanteilen erzeugt und abgegeben. Durch diese induzierten und an den Organismus abgegebenen Impulse werden im Organismus vorhandene Eigenimpulse optimal angeregt und unterstützt, wenn die Frequenz der von aussen aufgebrachten, magnetischen Wechselfelder mit diesen Eigenimpulsen synchron ist. Dadurch werden die angestrebten Reizungen des vegetativen Nervensystems unterstützt.

Ein wesentliches Merkmal der erfindungsgemäßen Vorrichtung liegt darin, dass die wenigstens eine Spule wenigstens zwei Lagen mit unterschiedlicher Wicklung aufweist. In Widerspruch zu herkömmlichen Spulen, auf denen der Draht in regelmässig übereinander liegenden Schichten gewickelt wird, wird die wenigstens eine Spule der erfindungsgemäßen Vorrichtung mit wenigstens zwei Lagen mit unterschiedlicher Wicklung ausgebildet. Dank diesem besonderen Wicklungsverfahren erhält das magnetische Wechselfeld besondere Eigenschaften im Zusammenhang mit dem Induzieren von Wirbelströmen in einem Organismus. Die unterschiedlichen Spulen dürfen nach demselben Muster gewickelt werden, was die Automatisierung oder die Reproduzierbarkeit der Herstellung der Spulen ermöglicht. Die Spulen sind beispielsweise ausgelegt für die Erzeugung von einem Magnetfeld bis zu 2 mT.

Durch die erfindungsgemäß vorgesehene Ausbildung der wenigstens einen Spule mit wenigstens zwei Lagen mit unterschiedlicher Wicklung können Spulen hergestellt bzw. bereitgestellt werden, welche wesentlich besser steile oberwellenhaltige Impulse erzeugen können. Derartige Spulen zur Erzeugung von steilen oberwellenhaltigen Impulsen zeigen deutlich bessere Wirkungen zur Behandlung insbesondere von Tinnitus als normal gewickelte Spulen.

Gemäß einem weiteren wesentlichen Merkmal ist erfindungsgemäß vorgesehen, dass die wenigstens eine Spule wenigstens eine Lage mit orthozyklischer Wicklung und wenigstens eine Lage mit schraubenförmiger Wicklung aufweist. Durch eine derart vorgesehene Ausbildung der Spule mit wenigstens einer Lage mit orthozyklischer Wicklung und wenigstens einer Lage mit schraubenförmiger Wicklung lassen sich die oben erwähnten steilen, oberwellenhaltigen Impulse entsprechend zuverlässig bei der im Bereich extrem niedriger Frequenzen betriebenen Spule erzielen.

Gemäß einem weiteren wesentlichen Merkmal ist erfindungsgemäß vorgesehen, dass die Vorrichtung zusätzlich Lautsprecher für die Tonausgabe umfasst. In einer Ausführungsvariante umfasst hierbei die erfindungsgemäße Vorrichtung zwei oder mehr Lautsprecher zur Kombination der Magnetfeldtherapie mit einer Tontherapie. Über die Lautsprecher werden Tinnitus-spezifische Tonsequenzen, beruhigende Musikstücke, Masker oder Noiser ausgegeben. Die Lautsprecher werden beispielsweise über eine Audiobüchse oder über eine kontaktlose Datenschnittstelle wie Bluetooth an einem Musikplayer angeschlossen. Die Steuerung der Musikwiedergabe erfolgt über ein Bluetooth fähiges Gerät, wie beispielsweise ein Smartphone. Die Laustärke und die Titel können direkt von der Anzeigevorrichtung angezeigt und von der Eingabevorrichtung angesteuert werden. Alternativ kann eine entsprechende Auswahl mit einer zusätzlichen, kleinen Fernsteuerung erfolgen.

Zur Verbesserung des angestrebten Effekts speziell ausgebildeter Impulse wird erfindungsgemäß vorgeschlagen, dass die wenigstens eine Spule eine Mehrzahl von benachbarten Lagen mit jeweils orthozyklischer und schraubenförmiger Wicklung aufweist.

Für eine besonders zuverlässige und präzise Positionierung der Spulen zur Erzeugung von magnetischen Wechselfeldern zum Induzieren von Wirbelströmen in einem Organismus an den für eine Behandlung insbesondere von Tinnitus gewünschten Stellen bzw. Positionen wird gemäß einer weiters bevorzugten Ausführungsform der Erfindung vorgeschlagen, dass die wenigstens eine Spule in bzw. an einem Kopfhörer, insbesondere in bzw. an einer Kopfhörermuschel angeordnet ist.

Zur Erzielung der zu induzierenden Wirbelströme an den gewünschten Stellen insbesondere zur Behandlung von Tinnitus wird darüber hinaus gemäß einer weiters bevorzugten Ausführungsform vorgeschlagen, dass jedem Ohr jeweils zwei Spulen an unterschiedlichen Höhenpositionen zugeordnet sind. Die jedem Ohr zuzuordnenden Spulen werden hierbei im Bereich des Cortex als auch des Mastoid angebracht bzw. positioniert, wobei die Cortex-Spulen beispielsweise im oberen Bereich der Hörmuschel eines Kopfhörers liegen, während die Spulen für den Mastoid an der unteren Seite der Hörmuschel des Kopfteils bzw. Kopfhörers insbesondere leicht schräg angebracht werden. Durch die erfindungsgemäß vorgeschlagene spezielle Anordnung der jedem Ohr zuzuordnenden Spulen an unterschiedlichen Höhenpositionen wird sichergestellt, dass das durch die elektromagnetischen Felder erzeugte Kraftfeld auch Zellen im Inneren des Körpers zur Erzielung der gewünschten Wirkung erreicht.

In diesem Zusammenhang wird gemäß einer weiters bevorzugten Ausführungsform vorgeschlagen, dass die jedem Ohr zugeordneten Spulen im Gegentakt betrieben werden. Durch einen Betrieb der jedem Ohr zugeordneten Spulen im Gegentakt wird durch die erzeugten elektromagnetischen Wechselfelder auf elektrische Ladungen eingewirkt und es werden derart ruhende Ladungen im Hörzentrum insbesondere im Takt ihrer eigenen Schwingung in Bewegung gesetzt. Durch den Betrieb der Spulen im Gegentakt werden das pulsierende elektromagnetische Wechselfeld überlagerte schwache Nachschwingungssignale erzeugt, welche therapeutisch bedeutsam sind, da sie insbesondere den Informationsaustausch zwischen den Zellen stimulieren können.

Eine besonders einfache und konstruktiv leicht herstellbare Ausführung der Erfindung ist bevorzugt derart ausgebildet, dass die Vorrichtung einen astabilen Multivibrator mit einem Transistor und wenigstens eine bipolare Eisenkernspule zur Abgabe der Wechselfelder aufweist. Die Erzeugung und Abgabe der magnetischen Wechselfelder lassen sich mit bekannten Elementen in relativ einfacher Form und kostengünstig herstellen. Die erfindungsgemäße Vorrichtung lässt sich insbesondere zur Behandlung von Ohrengeräuschen, Schlafstörungen, Kopfschmerzen, Migräne, Hals- und Rückenwirbelsyndromen, Nervosität und Durchblutungsstörungen verwenden. Dabei hat sich gezeigt, dass günstig wirkende Bereiche der magnetischen Wechselfelder mit einer einstellbaren, niedrigen Frequenz derart gewählt sind, dass die Frequenz unterhalb von 20 Hz, insbesondere zwischen 3 und 15 Hz, liegt. Allgemein wurde eine Unterteilung in zwei wesentliche behandlungsspezifische Bereiche gefunden, wobei ein Frequenzbereich zwischen 3 und 6 Hz allgemein beruhigend und krampflösend wirkt. Demgegenüber wirkt der Frequenzbereich zwischen 8 und 15 Hz im vorwiegend anregend, schmerzstillend und stabilisierend. Weiter kann davon ausgegangen werden, dass Frequenzen unter 8 Hz im wesentlichen Blutgefässerweiterungen bewirken, während Frequenzen über 12 Hz allgemein zu Blutgefässverengungen führen, wobei der oben angegebene, hohe Frequenzbereich nur angewandt werden sollte, wenn niedrigere Frequenzen wirkungslos sind.

Für eine weitere Verbesserung der Anpassung der einzustellenden Frequenz an die erwünschte Wirkung wird gemäß einer weiters bevorzugten Ausführungsform vorgeschlagen, dass ein insbesondere digitales Wobbeln der Frequenz um ± 50 %, bevorzugt ± 25 %, vorzugsweise ± 10 %, vorgesehen ist.

Für eine baulich einfache Konstruktion und insbesondere zur Ausbildung von Vorrichtungen, welche unabhängig von einer Netzspannungsversorgung betrieben werden können, wird darüber hinaus gemäß ein weiter bevorzugten Ausführungsform vorgeschlagen, dass als Spannungsversorgung eine Niederspannungs-Gleichspannungsquelle, insbesondere eine 9-Volt-Gleichspannungsquelle, vorgesehen ist. Derart lassen sich auch in einfacher Weise mobile und tragbare Geräte unabhängig von einer Netzversorgung durch Vorsehen einer Batterie als Gleichspannungsversorgung ausbilden, was insbesondere bei der Anwendung zur Behandlung von Tinnitus von Vorteil ist. Als Stromquelle lassen sich auch wiederladbare Akkumulatoren sehr gut einbauen.

Wie oben bereits angedeutet, lässt sich die erfindungsgemäße Vorrichtung zur Behandlung einer Vielzahl von Einsatzbereichen verwenden, wobei insbesondere zur Behandlung von Ohrengeräuschen bzw. Tinnitus, wie dies oben ausgeführt ist, die Vorrichtung in einem Kopfhörer aufgenommen ist, wobei in jedem Lautsprecher des Kopfhörers jeweils eine bipolare Eisenkernspule enthalten ist. Gegebenenfalls ist jedoch auch eine Ausbildung mit nur einer bipolaren Eisenkernspule denkbar, wenn beispielsweise nur in einem Ohr Ohrengeräusche auftreten. Eine erfindungsgemäße Vorrichtung lässt sich ohne weiteres in einem derartigen Kopfhörer, gegebenenfalls mit einem entsprechend kleinbauenden Zusatzgerät, verwirklichen, so dass insgesamt eine leicht anwendbare und einsetzbare Konstruktion erzielbar ist.

In einer Ausführungsvariante umfasst die erfindungsgemäße Vorrichtung eine Eingabevorrichtung und eine Anzeigevorrichtung. Wie oben bereits angedeutet, liegt ein wesentliches Merkmal der erfindungsgemäßen Vorrichtung in der Verfügbarkeit von einstellbaren, niedrigen Frequenzen zur Behandlung unterschiedlicher Indikationen sowie zur Erzielung einer bestmöglichen Übereinstimmung der magnetischen Wechselfelder und den der zu behandelnden Person innewohnenden Eigenimpulse. In diesem Zusammenhang wird vorgeschlagen, dass die Vorrichtung eine Mehrzahl von Schaltern bzw. Auswahlmittel zur Einstellung einer gewünschten Frequenz, Dauer der Therapie, Start/Stopp der Therapie aufweist. Derart lässt sich in einfacher Weise eine Anpassung an gewünschte Frequenzbereiche sowie einer möglichst genauen Übereinstimmung mit der Frequenz der Eigenimpulse der zu behandelnden Person erzielen.

In einer Ausführungsvariante sind die Mastoid- und Cortex-Spulen elektrisch einzeln angesteuert und so angeordnet, dass die jeweiligen Signale um einen beliebig wählbaren Wert zeitlich verschoben sind. Eine Verschiebung der Signale um eine halbe Periode zwischen der Mastoid-Spule und der Cortex-Spule auf der einen Kopfseite weist beispielsweise besondere Heilungswirkungen auf.

Für eine weitere Vereinfachung bei der Handhabbarkeit und Überwachung der erfindungsgemäßen Vorrichtung ist weiter bevorzugt vorgesehen, dass die Vorrichtung wenigstens eine Anzeige, insbesondere eine LCD-Anzeige oder eine Retina-Anzeige, aufweist. Zur weiteren Automatisierung des Einsatzes der erfindungsgemäßen Vorrichtung wird vorgeschlagen, dass die Vorrichtung einen Zeitgeber aufweist, wie dies einer weiteren bevorzugten Ausführungsform der Erfindung entspricht.

In einer Ausführungsvariante umfasst die erfindungsgemäße Vorrichtung Bereiche, die erwärmt werden können und in Kontakt mit oder in der Nähe von der Haut sind. Diese erwärmten Bereiche fördern zusätzlich die Durchblutung. Dadurch werden Verspannungen und Krämpfe im Ohrbereich ausgeglichen. Die Erwärmung erfolgt beispielsweise über Strahler im Infrarotbereich.

In einer Ausführungsvariante umfasst die erfindungsgemäße Vorrichtung eine Datenschnittstelle, beispielsweise eine drahtbehaftete Schnittstelle wie eine Audiobüchse für den Anschluss von einem Musikplayer oder von einem externen Kontroller für den Betrieb oder die Wartung der erfindungsgemäßen Vorrichtung, oder beispielsweise eine drahtlose Schnittstelle wie Bluetooth für den Anschluss von einem Smartphone. In einer Ausführungsvariante umfasst die erfindungsgemäße Vorrichtung eine Schnittstelle, mit der sich die Vorrichtung mit FitBit-Armbänder und Smart-Watches verbinden und ein Datenaustausch durchführen lässt. In einer Ausführungsvariante umfasst die erfindungsgemäße Vorrichtung eine Schnittstelle, mit der sich die Vorrichtung mit IoT (Internet der Dinge), d.h. dem Netzwerk verbinden lässt. Dazu wird ein RFID-Transponder in die Schaltung integriert.

In einer Ausführungsvariante umfasst die erfindungsgemäße Vorrichtung ein Speichermodul, beispielsweise eine Speicherkarte, beispielsweise eine SD-Karte zur Speicherung von Entspannungs- und Tinnitus heilender Musik, die über die Lautsprecher ausgegeben wird. Auf der Speicherkarte können ebenfalls Messdaten oder Protokolle über die Therapie zur späteren Auswertung abgelegt werden.

In einer Ausführungsvariante werden einige Funktionen der erfindungsgemäßen Vorrichtung in einen Basisteil ausgelagert. In einer solchen Ausführung umfasst der Kopfteil beispielsweise den Bügel, die Muscheln, die Applikatoren, die Lautsprecher, während der Basisteil die restlichen Komponenten, wie beispielsweise die Stromquelle, die Steuerung, die Anzeigevorrichtung, die Eingabevorrichtung, usw. umfasst. Diese Ausführungsvariante ist besonders vorteilhaft wenn gewisse Komponenten schwer, platzraubend und nur schwierig im Kopfteil integrierbar sind. Die Verbindung zwischen dem Kopf- und dem Basisteil erfolgt beispielsweise über ein Kabel oder über eine kontaktlose Schnittstelle wie Bluetooth. Gewisse Funktionen können ebenfalls im Sinne einer App in einem Smartphone integriert werden. In diesem Fall wird eine gerätespezifische App von einem App-Store geladen und auf dem Smartphone installiert. Als Eingabe- und Anzeigevorrichtung für die erfindungsgemäße Vorrichtung können diejenigen des Smartphones verwendet werden. Eine solche Ausführungsvariante führt zu Einsparungen für den Patienten. Die Kommunikation zwischen dem Smartphone und der erfindungsgemäßen Vorrichtung erfolgt über eine kontaktlose Schnittstelle, beispielsweise über Bluetooth, und ermöglicht einen Austausch von Daten in beiden Richtungen, nämlich vom Smartphone zur erfindungsgemäßen Vorrichtung für Steuerungszwecke, und von der erfindungsgemäßen Vorrichtung zum Smartphone für Diagnosezwecke.

Die Erfindung wird nachfolgend anhand von in der beiliegenden Zeichnung schematisch dargestellten Ausführungsbeispielen näher erläutert. In dieser zeigen:
Fig. 1 eine schematische Darstellung einer ersten Ausführungsform einer erfindungsgemäßen Vorrichtung;
Fig. 2 eine schematische Schaltungsanordnung der Ausführungsform gemäß Fig. 1;
Fig. 3 in einer zu Fig. 1 ähnlichen Darstellung eine abgewandelte Ausführungsform einer erfindungsgemäßen Vorrichtung; und
Fig. 4 in vergrößertem Maßstab eine teilweise schematische Darstellung einer Spule mit Lagen mit jeweils unterschiedlicher Wicklung.

Die erfindungsgemäße Vorrichtung wird anhand der Figuren nachstehend detailliert beschrieben.

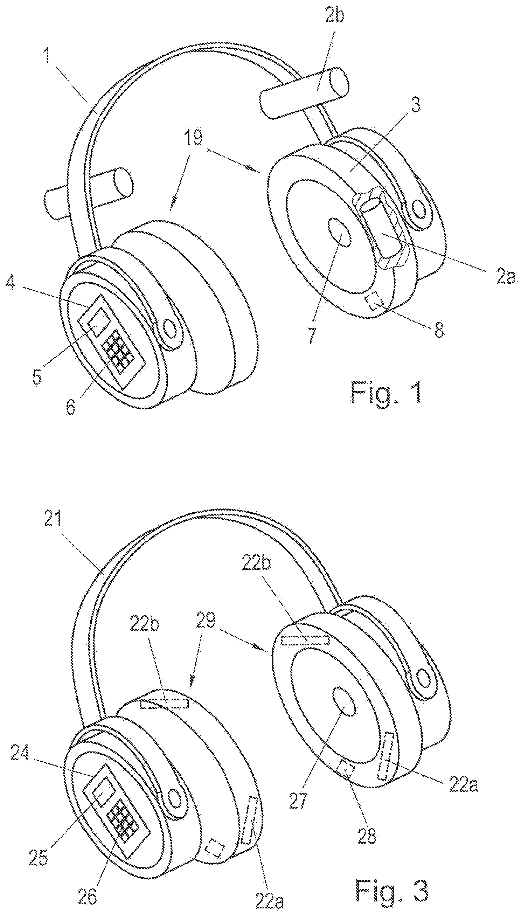

Die Vorrichtung gemäß Fig. 1 besteht beispielsweise aus einem Kopfhöher 1, auch Kopfteil genannt, mit einem gefederten Bügel und zwei Muscheln, die die Ohren überdecken. Für den Kopfteil wird hochwertiges Material verwendet, um den höchsten Komfort während der Therapie zu gewährleisten. Alle Materialen sind auf Hautverträglichkeit geprüft. In den Muscheln befinden sich jeweils ein Applikator 2, beispielsweise eine Eisenkernspule 2a. Diese sind für die Behandlung der Mastoidknochen vorgesehen und werden direkt im hinteren Bereich der Muschel eingegossen. Der Mastoidknochen ist ein gewölbter Knochen; er befindet sich unmittelbar hinter dem Ohr. Weitere Applikatoren, beispielsweise Eisenkernspulen 2b für den Cortex werden auch direkt in den Bügel 1 eingegossen, so dass sie während der Verwendung nicht verschoben werden können. Der Cortex (auch Kortex oder Grosshirnrinde genannt) ist die äußere, an Nervenzellen reiche Schicht des Grosshirns. In einer Ausführungsvariante werden die Eisenkernspulen in Reitern eingegossen, die am Bügel 1 angebracht und verschoben werden bis eine bestimmte Position des Cortex erreicht wird. Am Bügel 1 oder in den Muscheln versorgt befindet sich eine Stromquelle 3, beispielsweise eine Batterie oder ein wiederaufladbarer Akkumulator. In der Muschel oder im Bügel versorgt befindet sich eine Steuerung 4 und ein nicht dargestellter Signalverstärker für die Verstärkung der Applikatorsignale. Diese Elektronik überwacht gleichzeitig, ob die Applikatoren tatsächlich funktionieren. Dies geschieht durch die Messung des Stromflusses zum Kopfteil.

In Fig. 1 sind darüber hinaus eine Anzeige 5, eine Eingabevorrichtung 6, ein Lautsprecher 7 sowie Wärmepads bzw. Warmbereiche 8 in bzw. an jeder Hörmuschel 19 des Kopfhörers 1 angedeutet.

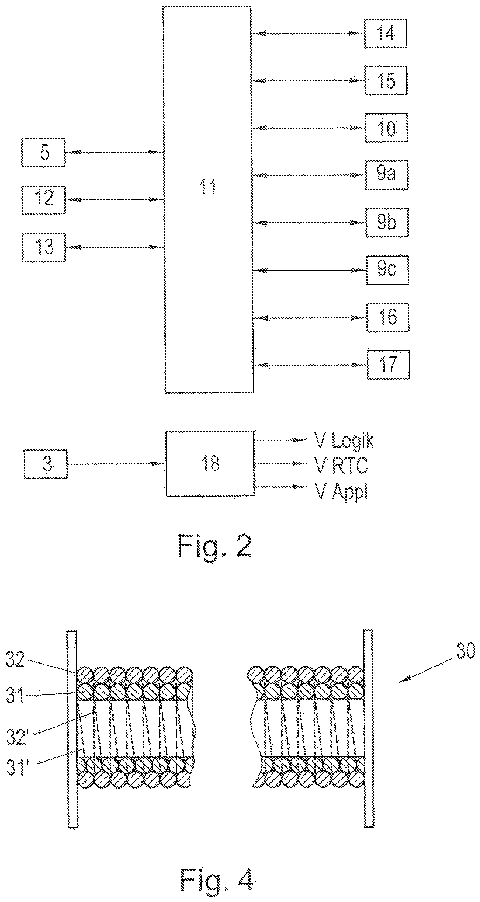

Die Vorrichtung umfasst eine Steuerung, die anhand der Fig. 2 nachstehend detailliert beschrieben wird. Die Elektronik der Steuerung soll auf modernster SMT-Technologie aufgebaut werden, die einen wesentlichen Kostenvorteil bei der Herstellung nach sich zieht. In einer Ausführungsvariante wird die Elektronik der Steuerung, inkl. Anzeige- und Eingabevorrichtung 6 im Kopfteil der erfindungsgemäßen Vorrichtung versorgt, so dass die gesamte erfindungsgemäße Vorrichtung eine völlig kabellose Therapie ermöglicht, was zum Abbau des Stresses beiträgt.

Die Steuerung 4 der erfindungsgemäßen Vorrichtung umfasst folgende Bestandteile:
- Controller 11: Beispielsweise vom Typ ATMEGA 16-P von Atmel mit 10 Kbyte internem Programmspeicher, 2K RAM und 28 Byte EEPROM. Der Programmspeicher ist als Flash ausgeführt und damit bis zu 10'000 Mal löschbar und beschreibbar.
- EEPROM 12: Im EEPROM werden folgende Daten abgelegt: bis 10 Kurvenformen ä 256 Byte (2'560 Byte), Betriebsstundenzähler (8 Byte), Patientendaten (256 Byte), Zeitprogramme (256 Byte). Damit ergeben sich als Gesamtzahl 3'080 Byte. Um für die Zukunft gerüstet zu sein wird hier ein serielles EEPROM mit 64 Kbit eingesetzt (z.B. AT24C64 von Atmel).
- RTC 13: Mit dem RTC (Echtzeituhr) ist es möglich, die genauen Uhrzeiten und Datum jeder einzelnen Behandlung in das Speichermodul 10 zu protokollieren und abzurufen.
- Reset 14: Supervisory Circuit, überwacht die Versorgungsspannung und liefert im Falle der Unterschreitung des definierten Grenzwertes ein definiertes RESET-Signal für die Prozessoreinheit. Dies sowohl im Power-Up Fall als auch bei Brown-Out. Weiters schaltet der Baustein im Off-Status des Geräts die Versorgung für den RTC Baustein.
- Clock 15: Taktfrequenz von 8 MHz für die Taktversorgung des Controllers 11. Aufgrund der notwendigen Genauigkeit der Taktfrequenz wird hier ein Keramikresonator verwendet.
- Display (Anzeigevorrichtung 5): Es wird ein Retina HD Display bevorzugt. Die Bedienung erfolgt mittels eigener App. Die komplette Steuerung 4, 24 der einzelnen Komponenten erfolgt ebenfalls mit eigenen Apps. Es ist aber auch möglich, fremde Tinnitus-Apps (Masker, Trainer oder Noiser) in die Bedienung einzubinden.
- USB-Buchse: Dieser Anschluss dient zum Laden des eingebauten Akkus. Weiter kann der USB-Anschluss für Programmupdates und Datenaustausch mit einem PC verwendet werden.
- D/A-Wandler 16: Der D/A-Wandler erzeugt das Ausgangssignal zum Applikator entsprechend einer im EEPROM abgelegten Kurvenform. Die Auflösung der Amplitude als auch der Frequenz soll bei 256 liegen (= zeitdiskrete und amplitudendiskrete Ausgabe) .
- PWM-Output: In diesem Fall wird vom Controller 11 ein PWM-Signal geliefert. Amplitudendefinition über DA-Wandler.
- Output Amplifier 17: Verstärkt das vom DA-Converter gelieferte Applikatorsignal auf die maximale Amplitude und überwacht gleichzeitig, ob die Applikatoren (Kopfteil) tatsächlich funktionieren. Dies geschieht durch eine Messung des Stromflusses (Signal APPL OK) zum Kopfteil, der über den internen Analog - Digitalwandler des Controllers 11 gemessen und entsprechend bewertet wird. Auf Basis dieser Messung ist eine Bewertung der Applikatorfunktion möglich. Es werden die von der App gewählten Applikatoren mit den gleichen Signalen angesprochen.
- Bluetooth: Die Steuerung 4, 24 verfügt nun über ein Bluetooth-Modul. Es ist jetzt möglich mittels Smartphone oder Bluetooth-fähigen Player Musik oder die Tinnitus-App in das Tinnitusgerät über das Kopfteil auszugeben. Die Steuerung 4, 24 kann über das Smartphone erfolgen.
- Audiobuchse: Über die Audiobuchse kann auch ein nichtfähiges Smartphone oder Player angeschlossen und über das Kopfteil die Tinnitus-Trainer, Masker oder Noiser ausgegeben werden.
- SD Memory Card: Mit einer SD Speicherkarte ist es möglich, eigene Entspannungs- oder Tinnitusmusik in das Gerät einzuspielen und über den Kopfteil auszugeben.
- Audio: Hierfür wird der Audiobaustein (MS6331G) verwendet. Die Lautstärke und die Titel können direkt vom Display 5, 25 (Anzeigevorrichtung) oder Smartphone angesteuert werden.
- Spannungsaufbereitung 18: Das Gerät wird mit einem internen Akku versorgt. Es werden folgende Spannungen aufbereitet: VLogik: Versorgungsspannung für den Logikteil und Bluetooth-Modul 3.3 V. VAppl: Applikatorspannung für den Output-Amplifier. VRTC: Backup-Spannung für RTC 13 und Echtzeit. Im Weiteren überwacht die Spannungsaufbereitung 18 auch noch die Akkuspannung (LOWBAT) und führt diese dem AD-Wandler des Controllers 11 zu. Der misst die Spannung, damit frühzeitig auf ein notwendiges Laden hingewiesen werden kann. Die Entwicklung wird unter Berücksichtigung Strom sparender Komponenten ausgeführt.
- Infrarot Wärme: Infrarot Wärmepads 8 werden in die Earpads (Muschel) eingearbeitet. Diese fördern zusätzlich die Durchblutung. Dadurch werden auch Verspannungen und Krämpfe im Ohrbereich ausgeglichen.
- Applikatoren-Spulen: Diese Spulen für den Mastoidknochen werden direkt im hinteren Bereich der Muschel eingegossen. Die Spulen für den Cortex werden auch direkt in den Bügel 1, 21 eingegossen, so dass sie während der Verwendung nicht verschoben werden können. Es wird ein hochwertiges Kopfteil in Verwendung gebracht um den höchsten Komfort während der Therapie zu gewährleisten. Alle Materialen sind auf Hautunverträglichkeiten geprüft.

Die Anwendung umfasst folgende Funktionen:
- Applikatorsignal: Zehn Kurvenformen sind speicherbar, gleichzeitig, überlagerbar oder auch kombinierbar. Auswahl erfolgt mithilfe des Displays.
- Maximale Frequenz des Applikators 200 Hz.
- Wichtiger Teile der Frequenz ist ein zusätzliches Wobbeln.
- Diese Frequenzen werden in verschiedenen Programmen ausgewählt.
- Betriebsstundenzähler von 0 - 9999 (Abspeicherung und Anzeige in 1/10 Stunden) und Anzeige am Display.
- Zustandsüberwachung Akku (LOWBAT) und Anzeige im Display.
- Zustandsüberwachung Applikator (APPLOK) und Anzeige im Display.
- Verbundene Bluetooth-Anzeige (DL-Link) und Anzeige im Display.
- SD Memory Card - Titel einzeln wählbar und steuerbar. Anzeige im Display.
- Lautstärkeregelung für die Audioausgabe lauter/leiser («/») und Anzeige im Display.
- Applikatoren für Cortex und/oder Mastoid können einzeln ein-/ausgeschaltet werden und angezeigt im Display.
- Menüführung und Apps am Display.
- Zeitüberwachung und automatische Abschaltung des Displays (der Hintergrundbeleuchtung) und/oder des Controllers 11.
- BUSY-Anzeige am Display, um den Zustand "BEREIT" am Gerät anzuzeigen.
- Abspeicherung von Daten ins EEPROM wie z.B. Betriebsstundenzähler, Anzahl der gestarteten Behandlungen.
- USB-Buchse, um Programmänderungen und Laden des Akkus vorzunehmen.

Die Verarbeitung der Patientendaten kann über ein Upload bewerkstelligt werden. Die Daten können dann mittels PC und einer gesonderten Software verarbeitet werden.

In einer Ausführungsvariante bestehen die Spulen aus einem Eisenkern. Das Ausmass des Eisenkerns beträgt eine Länge von 30 mm bis 60 mm, insbesondere 50 mm bis 60 mm und einen Durchmesser von 6 mm. Die Spule umfasst 600 bis 650 Wicklungen aus einem Kupferdraht mit einem Durchmesser von 0.2 mm.

Bei der Ausführungsform gemäß Fig. 3 ist wiederum eine Mehrzahl von Spulen in einem Kopfhörer 21 bzw. insbesondere den Kopfhörermuscheln 29 integriert, wobei ersichtlich ist, dass bei der in Fig. 3 dargestellten Ausführungsform sowohl die Spulen 22a für den Mastoid als auch die Spulen 22b für den Cortex jeweils an unterschiedlichen Höhenpositionen in den Kopfhörermuscheln 29 integriert sind.

Ähnlich wie bei der Ausführungsform gemäß Fig. 1 sind zusätzliche Elemente, wie eine Steuerung 24, eine Anzeige 25, eine Eingabevorrichtung 26, Lautsprecher 27 sowie zu wärmende Bereiche bzw. Wärmepads 28 ebenfalls in die Kopfhörermuschel 29 integriert und schematisch angedeutet.

In Fig. 4 ist schematisch und in vergrößerter, nicht maßstabsgetreuer Darstellung eine Spule 30 teilweise im Schnitt gezeigt, wobei ersichtlich ist, dass eine mit 31 bezeichnete erste Wicklungslage schraubenförmig gewickelt ist, wie dies durch den strichliert angedeuteten Verlauf 31' der Drähte dieser Lage 31 angedeutet ist.

Anschließend ist eine Lage 32 angedeutet, deren Drähte orthozyklisch gewickelt sind, wie dies durch den schematisch angedeuteten Verlauf 32' gezeigt ist, welche im Wesentlichen normal auf die Spulenachse gegenüber der schrägen bzw. geneigten Anordnung der schraubenförmigen Wicklung 31 bzw. 31' stehen.

Durch die Anordnung von Lagen 31, 32 mit unterschiedlicher Wicklung lassen sich insbesondere Felder mit steilen oberwellenhaltigen Impulsen im Gegensatz zu Spulen mit gleichmäßiger bzw. einheitlicher Wicklung erzielen, wobei sich derartige Fälle insbesondere in einem Gegentaktbetrieb der jedem Ohr zugeordneten Spulen 2a und 2b bzw. 22a und 22b erzielen lassen.

Entsprechend der zu erzielenden Feldstärke ist die in Fig. 4 lediglich schematisch angedeutete Spule 30, welche sowohl für die Cortex-Spulen 2b und 22b als auch die Mastoid-Spulen 2a bzw. 22a Verwendung finden kann, mit einer entsprechend großen Anzahl von Wicklungen und Wicklungslagen versehen, welche wenigstens teilweise unterschiedliche Wicklungen beinhalten.

### Bezugszeichenliste

- 1, 21: Bügel
- 2a, 22a: Spule Mastoid
- 2b, 22b: Spule Cortex
- 3: Stromquelle
- 4, 24: Steuerung
- 5, 25: Anzeigevorrichtung (Display, HD Retina-Display)
- 6, 26: Eingabevorrichtung (Touchscreen, Spracherkennung)
- 7, 27: Lautsprecher
- 8, 28: Warmbereich bzw. Wärmepad
- 9a: Serielle Datenschnittstelle (RS232)
- 9b: Audio Schnittstelle
- 9c: Datenschnittstelle (Bluetooth)
- 10: Speichermodul
- 11: Controller
- 12: Speicher (EEPROM)
- 13: RTC (Echtzeituhr)
- 14: Reset
- 15: Clock (Uhr)
- 16: D/A Wandler
- 17: Ausgangsverstärker (Output Amplifier)
- 18: Spannungsaufbereitung
- 19, 29: Kopfhörermuschel
- 30: Spule
- 31, 31': schraubenförmige Wicklung
- 32, 32': orthozyklische Wicklung

## Patentansprüche

1. Vorrichtung zur Erzeugung von magnetischen Wechselfeldern zum Induzieren von Wirbelströmen in einem Organismus zur Behandlung insbesondere von Tinnitus, umfassend
- einen Kopfteil (1, 21),
- eine Stromquelle (3),
- eine Steuerung (4, 24),
- eine oder mehrere Spulen (2a, 2b, 22a, 22b),
- einen Lautsprecher für die Tonausgabe, **dadurch gekennzeichnet, dass** die wenigstens eine Spule wenigstens zwei Lagen mit unterschiedlicher Wicklung aufweist, dass die wenigstens eine Spule wenigstens eine Lage mit orthozyklischer Wicklung und wenigstens eine Lage mit schraubenförmiger Wicklung aufweist und dass die wenigstens eine Spule eine Mehrzahl von benachbarten Lagen mit jeweils orthozyklischer und schraubenförmiger Wicklung aufweist.

2. Vorrichtung nach Anspruch 1, **dadurch gekennzeichnet, dass** die wenigstens eine Spule (2a, 2b, 22a, 22b, 30) in bzw. an einem Kopfhörer (1, 21), insbesondere in bzw. an einer Kopfhörermuschel (19, 29) angeordnet ist.

3. Vorrichtung nach einem der Ansprüche 1 bis 2, **dadurch gekennzeichnet, dass** jedem Ohr jeweils zwei Spulen (2a, 2b, 22a, 22b) an unterschiedlichen Höhenpositionen zugeordnet sind.

4. Vorrichtung nach Anspruch 3, **dadurch gekennzeichnet, dass** die jedem Ohr zugeordneten Spulen (2a, 2b, 22a, 22b) im Gegentakt betrieben werden.

5. Vorrichtung nach einem der vorherigen Ansprüche, **dadurch gekennzeichnet, dass** die Vorrichtung einen astabilen Multivibrator umfassend zumindest einen Transistor und wenigstens eine bipolare Eisenkernspule zur Abgabe der Wechselfelder aufweist.

6. Vorrichtung nach einem der vorherigen Ansprüche, **dadurch gekennzeichnet, dass** die Frequenz unterhalb von 20 Hz, insbesondere zwischen 3 und 15 Hz, gewählt ist.

7. Vorrichtung nach einem der vorherigen Ansprüche, **dadurch gekennzeichnet, dass** ein insbesondere digitales Wobbeln der Frequenz um ± 50 %, bevorzugt ± 25 %, vorzugsweise ± 10 %, vorgesehen ist.

8. Vorrichtung nach einem der vorherigen Ansprüche, **dadurch gekennzeichnet, dass** als Spannungsversorgung (3) eine Niederspannungs-Gleichspannungsquelle, insbesondere eine 9-Volt-Gleichspannungsquelle, vorgesehen ist.

9. Vorrichtung nach einem der vorherigen Ansprüche, **dadurch gekennzeichnet, dass** die Vorrichtung wenigstens eine Anzeige (5, 25), insbesondere eine LCD-Anzeige, aufweist.

10. Vorrichtung nach einem der vorherigen Ansprüche, **dadurch gekennzeichnet, dass** die Vorrichtung zusätzlich eine Eingabevorrichtung (6, 26) und eine Anzeigevorrichtung (5, 25) umfasst.

11. Vorrichtung nach einem der vorherigen Ansprüche, **dadurch gekennzeichnet, dass** die Vorrichtung einen oder mehrere Warmbereich(e) (8, 28) umfasst.

12. Vorrichtung nach einem der vorherigen Ansprüche, **dadurch gekennzeichnet, dass** die Vorrichtung eine Datenschnittstelle (9a, 9b) umfasst.

13. Vorrichtung nach einem der vorherigen Ansprüche, **dadurch gekennzeichnet, dass** die Vorrichtung ein Speichermodul (10) umfasst.

## Claims

1. A device for generating alternating magnetic fields for inducing eddy currents in an organism for treating tinnitus in particular, comprising
- a head part (1, 21),
- a current source (3),
- a controller (4, 24),
- one or more coils (2a, 2b, 22a, 22b),
- a loudspeaker for sound output, **characterized in that** the at least one coil comprises at least two layers with different windings, that the at least one coil comprises at least one layer with an orthocyclic winding and at least one layer with a helical winding and that the at least one coil comprises a plurality of adjacent layers each with orthocyclic and helical windings.

2. A device according to claim 1, **characterized in that** that the at least one coil (2a, 2b, 22a, 22b, 30) is disposed in or on a headphone (1, 21), in particular in or on an ear cup (19, 29) of a headphone.

3. A device according to any one of claims 1 to 2, **characterized in that** two coils (2a, 2b, 22a, 22b, 30) are each assigned to each ear on different height levels.

4. A device according to claim 3, **characterized in that** the coils (2a, 2b, 22a, 22b, 30) assigned to each ear are operated in the push-pull mode.

5. A device according to any one of the preceding claims, **characterized in that** the device comprises an astable multivibrator with at least one transistor, and at least one bipolar iron-core coil for emitting the alternating fields.

6. A device according to any one of the preceding claims, **characterized in that** the frequency is selected below 20 Hz, in particular between 3 and 15 Hz.

7. A device according to any one of the preceding claims, **characterized in that**, in particular digital, wobbling of the frequency by + 50%, preferably + 25%, preferably + 10%, is provided.

8. A device according to any one of the preceding claims, **characterized in that** that a low-voltage direct voltage source, in particular a 9 volt direct voltage source, is provided as a voltage supply (3).

9. A device according to any one of the preceding claims, **characterized in that** the device comprises at least one display (5, 25), in particular an LCD display.

10. A device according to any one of the preceding claims, **characterized in that** the device additionally comprises an input device (6, 26) and a display device (5, 25).

11. A device according to any one of the preceding claims, **characterized in that** the device comprises one or several heated area(s) (8, 28).

12. A device according to any one of the preceding claims, **characterized in that** the device comprises a data interface (9a, 9b).

13. A device according to any one of the preceding claims, **characterized in that** the device comprises a memory module (10) .

## Revendications

1. Un dispositif servant à générer des champs magnétiques alternatifs pour induire des courants de Foucault dans un organisme pour le traitement notamment des acouphènes, comprenant
- une partie de tête (1, 21),
- une source de courant (3),
- une commande (4, 24),
- une ou plusieurs bobines (2a, 2b, 22a, 22b),
- un haut-parleur pour la sortie du son, **caractérisé en ce que** ladite au moins une bobine comprend au moins deux couches avec des enroulements différents, **en ce que** ladite au moins une bobine comprend au moins une couche avec un enroulement orthocyclique et au moins une couche avec un enroulement hélicoïdal, et **en ce que** ladite au moins une bobine comprend une pluralité de couches adjacentes avec respectivement un enroulement orthocyclique et un enroulement hélicoïdal.

2. Dispositif selon la revendication 1, **caractérisé en ce que** ladite au moins une bobine (2a, 2b, 22a, 22b, 30) est agencée dans ou sur un casque d'écoute (1, 21), notamment dans ou sur une coque (19, 29) de casque d'écoute.

3. Dispositif selon l'une des revendications 1 à 2, **caractérisé en ce que** deux bobines (2a, 2b, 22a, 22b) sont respectivement associées à chaque oreille en des positions en hauteur différentes.

4. Dispositif selon la revendication 3, **caractérisé en ce que** les bobines (2a, 2b, 22a, 22b) associées à chaque oreille fonctionnent en va-et-vient.

5. Dispositif selon l'une des revendications précédentes, **caractérisé en ce que** le dispositif comporte un multivibrateur astable comprenant au moins un transistor et au moins une bobine bipolaire à noyau de fer pour délivrer les champs alternatifs.

6. Dispositif selon l'une des revendications précédentes, **caractérisé en ce que** la fréquence est choisie inférieure à 20 Hz, notamment entre 3 et 15 Hz.

7. Dispositif selon l'une des revendications précédentes, **caractérisé en ce qu'**il est prévu une oscillation, notamment numérique, de la fréquence de ± 50 %, de façon préférée de ± 25 %, de préférence de ± 10 %.

8. Dispositif selon l'une des revendications précédentes, **caractérisé en ce qu'**il est prévu, en tant qu'alimentation en tension (3), une source de tension continue à basse tension, en particulier une source de tension continue de 9 volts.

9. Dispositif selon l'une des revendications précédentes, **caractérisé en ce que** le dispositif comporte au moins un afficheur (5, 25), en particulier un afficheur LCD.

10. Dispositif selon l'une des revendications précédentes, **caractérisé en ce que** le dispositif comprend en outre un dispositif d'entrée (6, 26) et un dispositif d'affichage (5, 25).

11. Dispositif selon l'une des revendications précédentes, **caractérisé en ce que** le dispositif comprend une ou plusieurs zone(s) chaude(s) (8, 28).

12. Dispositif selon l'une des revendications précédentes, **caractérisé en ce que** le dispositif comprend une interface de données (9a, 9b).

13. Dispositif selon l'une des revendications précédentes, **caractérisé en ce que** le dispositif comprend un module de stockage (10).
